# EUROPÄISCHE PATENTSCHRIFT

(11) **EP 1 690 967 B1**
(45) Veröffentlichungstag und Bekanntmachung des Hinweises auf die Patenterteilung: **17.09.2008**
(21) Anmeldenummer: 05003001.4
(22) Anmeldetag: 12.02.2005
(51) Int. Cl.: D04B 21/02, A44B 18/00

(54) **Verbundstoffelement für einen Klettverschluss, insbesondere einen Windelverschluss, und Verfahren zu seiner Herstellung**
Composite element for a contact fastener, in particular for a nappy closure, and method of making same
Matériau composite pour une fermeture auto-grippante, notamment pour la fermeture d'un article absorbant, et procédé de production dudit matériau

(43) Veröffentlichungstag der Anmeldung: 16.08.2006
(73) Patentinhaber: Nordenia Deutschland Gronau GmbH, 48599 Gronau (DE)
(72) Erfinder: Baldauf, Georg, 48366 Laer (DE)
(74) Vertreter: Albrecht, Rainer Harald

(56) Entgegenhaltungen:
- WO-A-20/04050970
- US-A- 4 643 932
- US-A- 5 957 908

## Beschreibung

Die Erfindung betrifft ein Verbundstoffelement für einen Klettverschluss, insbesondere einen Windelverschluss, mit einer Trägerfolie und einem auf die Trägerfolie aufkaschierten textilen Material, welches eine zur Verbindung mit Kletthaken geeignete Oberflächenstruktur aufweist, wobei die Trägerfolie und das textile Material in einem Randbereich, der sich entlang den Rändern des Verbundstoffelementes erstreckt und die Form eines Rahmens aufweist, verbunden sind und wobei im Bereich innerhalb dieses Rahmens eine nicht vollflächige Verbindung zwischen der Trägerfolie und dem textilen Material vorgesehen ist.

Das Verbundstoffelement bildet den weiblichen Teil eines Klettverschlusses. Bei der Verwendung an Windeln wird das Verbundstoffelement auf dem vorderen Bündchenbereich der Windel angebracht. Ein Verschlussband, das seitlich an der Windel befestigt ist und an seinem freien Ende Kletthaken aufweist, vervollständigt einen Klettverschluss. Klettverschlüsse können mehrfach geöffnet und verschlossen werden, ohne dass dadurch die Funktionalität des Verschlusses leidet. Im Gegensatz zu Klebeverschlüssen sind Klettverschlüsse unempfindlich gegenüber Kontakt mit Hautcremes oder Puder.

An ein Verbundstoffelement für einen Klettverschluss an einem Wegwerfprodukt, z. B. Babywindeln, werden mehrere Anforderungen gestellt. Das textile Material soll ein möglichst geringes Flächengewicht aufweisen, damit es kostengünstig gefertigt werden kann. Es soll durchscheinend sein, damit die zumeist bedruckte Oberfläche, im besonderen auch auf die Trägerfolie gedruckte Rapportmarken für die weitere Verarbeitung des Verbundstoffes, sichtbar sind. Ferner muss das textile Material trotz eines geringen Flächengewichtes eine ausreichende Verhakung mit Kletthaken des zugeordneten Verschlussbandes gewährleisten. Erforderlich ist eine ausreichende Zahl von freibeweglichen Schlaufen und Fasern, deren Funktion durch eine Verklebung von Trägerfolie und textilem Material nicht beeinträchtigt werden darf. Um eine funktionssichere Verklebung des textilen Materials auf der Trägerfolie zu gewährleisten, muss ein ausreichend dicker Klebstofffilm aufgetragen werden. Wenn das textile Material und die Trägerfolie in einem Kaschierwerk mittels eines Walzenpaares aufeinander gepresst werden, sinken die Fasern des textilen Materials in den Klebstofffilm ein und werden von dem Klebstofffilm umschlossen. Nach Aushärtung des Klebstofffilmes sind die Garne des Textils zwar sicher auf der Trägerfolie verankert, jedoch bergen große Klebstoffmengen die Gefahr, dass die Schlaufen und Fasern, die für die Funktion des Klettverschlusses nötig sind, mit verklebt werden und dadurch ihre Funktionsfähigkeit verlieren. Dies macht sich in einer unzureichenden Klettwirkung bemerkbar.

Die Druckschrift US 4 643 936 beschreibt ein streifenförmiges Verbundstoffelement für einen Klettverschluss mit den eingangs beschriebenen Merkmalen. Die Trägerfolie und das textile Material des streifenförmigen Verbundstoffelementes sind randseitig und entlang einer zick-zack-förmigen Linie durch ein Hochfrequenz-Schweißverfahren miteinander verschmolzen.

Die Druckschrift US 5 957 908 beschreibt den Aufbau einer Windel mit Klettverschlüssen. Die Klettverschlüsse weisen jeweils einen Streifen mit Kletthaken und einen Streifen eines textilen Materials auf. Die Streifen sind durch musterförmig angeordnete Verbindungsabschnitte mit einer Trägerfolie verbunden. Bei einer wiederholten Betätigung des Klettverschlusses können sich die randseitig nicht durchgehend mit der Trägerfolie verbundenen Streifen lösen.

Aus EP 0 777 006 B1 ist ein Verbundstoff für Klettverschlüsse mit einem auf eine Trägerfolie aufkaschierten textilen Material bekannt. Das textile Material besteht aus einem Gelege aus Kett- und Schussfäden und wirktechnisch mit dem Gelege verbundenen Schlaufen. Das textile Material ist mit der Trägerfolie verklebt. Die Schlaufen sind so groß bemessen, dass sie auf den von dem Grundgelege gebildeten Maschen aufliegen. Durch die Bemessung der Schlaufen soll erreicht werden, dass die Schlaufen nicht mit Klebstoff in Verbindung kommen und ihre Funktionsfähigkeit behalten. Das vorstehend erläuterte Problem, bei Verwendung eines offenen textilen Materials sowohl eine gute Klettwirkung als auch eine hohe Verbundfestigkeit zwischen Trägerfolie und textilem Material sicherzustellen, ist jedoch noch nicht in vollem Umfange gelöst. Insbesondere die Verbindung zwischen Trägerfolie und textilem Material ist noch verbesserungsbedürftig.

Der Erfindung liegt die Aufgabe zugrunde, in einem Verbundstoffelement für einen Klettverschluss die Verbindung zwischen Trägerfolie und textilem Material so auszubilden, dass sowohl eine hohe Verbundfestigkeit als auch eine gute Klettwirkung erreicht werden.

Die Aufgabe wird erfindungsgemäß dadurch gelöst, dass die Trägerfolie und das textile Material miteinander verklebt sind, wobei der Klebstoff zwischen der Trägerfolie und dem textilen Material in dem rahmenförmigen Randbereich flächig aufgetragen ist und einen Klebstoffrahmen bildet und wobei der Klebstoff in dem von dem Klebstoffrahmen umschlossenen Bereich in einem Klebstoffmuster angeordnet ist, welches sich aus Klebeflächen und klebstofffreien Bereichen zusammensetzt. Durch die flächige Verbindung, die sich in Form eines Rahmens entlang der Ränder des Verbundstoffelementes erstreckt, wird am Rand des Verbundstoffelementes, wo einerseits beim Öffnen des Klettverschlusses die größten Kräfte angreifen und andererseits offene Enden des textilen Materials vorliegen, ein Ausfransen des textilen Materials und ein Abreißen des textilen Materials von der Trägerfolie bei der Betätigung des Klettverschlusses verhindert. Vorzugsweise weist der flächig verbundene Randbereich eine Breite von 5 mm bis 30 mm auf. Der Bereich innerhalb des Rahmens weist vorzugsweise eine Länge zwischen 20 mm und 100 mm und eine Breite zwischen 50 mm und 500 mm auf.

Vorzugsweise beträgt die Klebstofffläche innerhalb des von dem Klebstoffrahmen umschlossenen Bereiches 20 % bis 80 % bezogen auf die von dem Klebstoffrahmen umschlossene Fläche. Im Bereich der Klebeflächen kann eine große Klebstoffmenge gewählt werden, die eine sichere, kraftschlüssige Verbindung zwischen textilem Material und der Trägerfolie sicherstellt, wobei auch eine weitgehende Durchtränkung des textilen Materials an den Klebeflächen in Kauf genommen werden kann. Erfindungsgemäß liegen Trägerfolie und das textile Material auf einem Teil der Kaschierfläche lose aufeinander auf. Diese klebstofffreien Bereiche bleiben für die Verbindung mit den Kletthaken voll erhalten. Die Kletthaken können sich dort gut mit dem textilen Material verhaken, da sie tief in das textile Material eintauchen können. Zusätzlich zu den oberflächlichen Schlaufen und Fasern stehen in den klebstofffreien Bereichen auch die das Grundgelege des textilen Materials bildenden Garne für eine Verankerung mit Kletthaken zur Verfügung. Die Kletthaken können hinter die Garne des Grundgeleges eingreifen. Der Verbundstoff erhält hierdurch eine Klettwirkung, die signifikant höher ist als bei einer vollflächigen Verklebung von textilem Material und Trägerfolie.

Die erfindungsgemäße Lehre besteht im Kern darin, dass die zur Verfügung stehende Verbindungsfläche des Kaschierverbundes aufgeteilt wird in Klebeflächen, die aus einem Rahmen und einem Muster von Klebstoff gebildet werden und einen sicheren und dauerhaften Verbund zwischen Trägerfolie und textilem Material herstellen, und klebstofffreien Bereichen, in denen eine gute Klettwirkung zwischen textilem Material und Kletthaken gewährleistet ist.

Die erfindungsgemäße Lehre kann mit einer Vielfalt von Klebstoffmustern innerhalb des Klebstoffrahmens am Rand des Verbundstoffelementes realisiert werden. So umfasst die Erfindung eine punktförmige Verklebung der Trägerfolie und des textilen Materials. Ferner besteht die Möglichkeit, dass der Klebstoff ein Muster aus parallelen oder sich kreuzenden Streifen bildet, wobei die Streifen als gerade Linien oder wellenförmige Linien ausgebildet sein können. Eine weitere bevorzugte Ausführung der Erfindung sieht vor, dass der Klebstoff ein Muster mit einer zellenförmigen Struktur bildet. Zellenstrukturen mit offenen, klebstofffreien Zellen innerhalb des Klebstoffrahmens erweisen sich als besonders wirksam. Das textile Material, welches zweckmäßig ein Flächengewicht zwischen 5 g/m² und 50 g/m² aufweist, besteht gemäß einer bevorzugten Ausführung der Erfindung aus einer Kettwirkware, welche ein Grundgelege aus Filamentgarnen und mit dem Grundgelege wirktechnisch verbundene Schlaufen aufweist. Dieses Material ist durchscheinend und sehr luftdurchlässig. Die erfindungsgemäße Lehre ermöglicht die Verarbeitung von textilen Materialien mit geringem Flächengewicht zu Bestandteilen funktioneller Windelverschlüsse. Das textile Material kann aus Monofil- und/oder Multifilamentgarnen, beispielsweise aus Polypropylen, Polyester, Polyamid oder anderen textiltechnisch verarbeitbaren Kunststoffen bestehen.

Die Trägerfolie weist gemäß einer bevorzugten Ausführung ebenfalls ein Flächengewicht zwischen 5 g/m² und 50 g/m² auf. Es können Monofolien ebenso wie mehrschichtige coextrudierte oder kaschierte Folien verwendet werden. Geeignet sind beispielsweise Folien aus Polyethylen, Polypropylen, Polyester, Polyamid, sowie Mischungen und Copolymerisate auf Basis dieser Polymere. Vorzugsweise werden Trägerfolien verwendet, deren Oberfläche im Schöndruck bedruckt werden kann. Es können bedruckte und/oder geprägte Trägerfolien eingesetzt werden. Durch die Verwendung einer elastischen Folie, die bei der Laminierung gedehnt ist, kann eine Wölbung des textilen Materials erreicht werden, was zu einer weiteren Verbesserung der Klettwirkung führen kann.

Für die Verklebung von textilem Material und Trägerfolie eignen sich prinzipiell alle auf dem Gebiet der Kaschierfolien verwendeten Klebstoffe. Bevorzugt sind Schmelzklebstoffe auf der Basis PAO, EVA, SBS, SIS, reaktive Polyurethanklebstoffe, Acrylatklebstoffe sowie auch strahlenhärtende Klebstoffe.

Gegenstand der Erfindung ist auch ein Verfahren nach Anspruch 11 zur Herstellung der beschriebenen Verbundstoffelemente. Bevorzugte Ausgestaltungen des erfindungsgemäßen Verfahrens sind in den Ansprüchen 12 und 13 beschrieben.

Im Folgenden wird die Erfindung anhand einer lediglich ein Ausführungsbeispiel darstellenden Zeichnung erläutert. Es zeigen schematisch:
- **Fig. 1**: eine Draufsicht auf ein Verbundstoffelement und dessen Schichten,
- **Fig. 2**: einen Schnitt durch das Verbundstoffelement nach Fig. 1 entlang der Linie A-A,
- **Fig. 3a bis 3d**: Klebstoffmuster, die eine Verbindungsschicht zwischen Trägerfolie und dem textilen Material des in den Fig. 1 und 2 dargestellten Verbundstoffelementes bilden,
- **Fig. 4**: Verfahren zur Herstellung des Verbundstoffes und
- **Fig. 5**: einen Ausschnitt aus einer Folienbahn mit aufgebrachtem Klebstoff vor der Verbindung mit dem textilen Material.

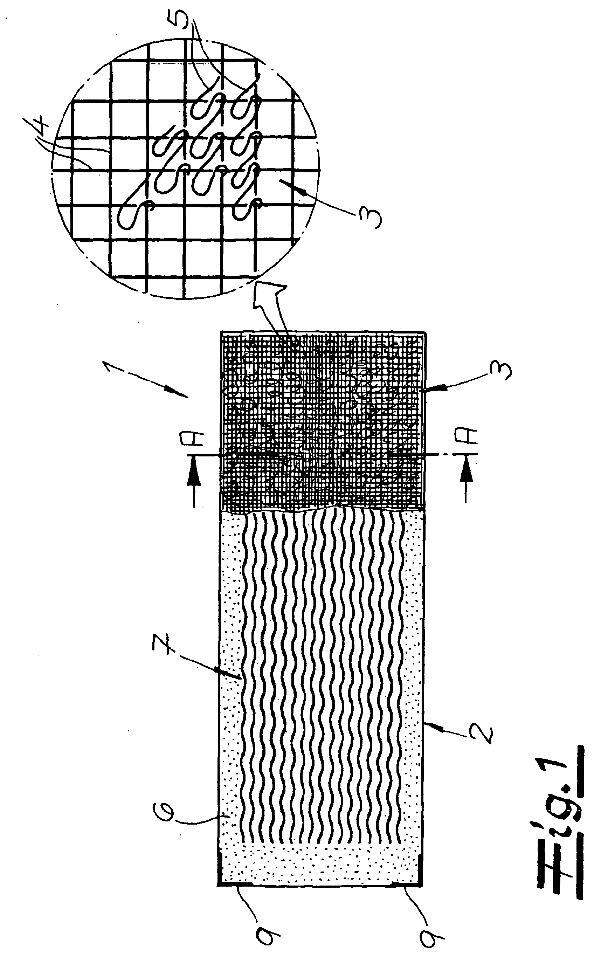

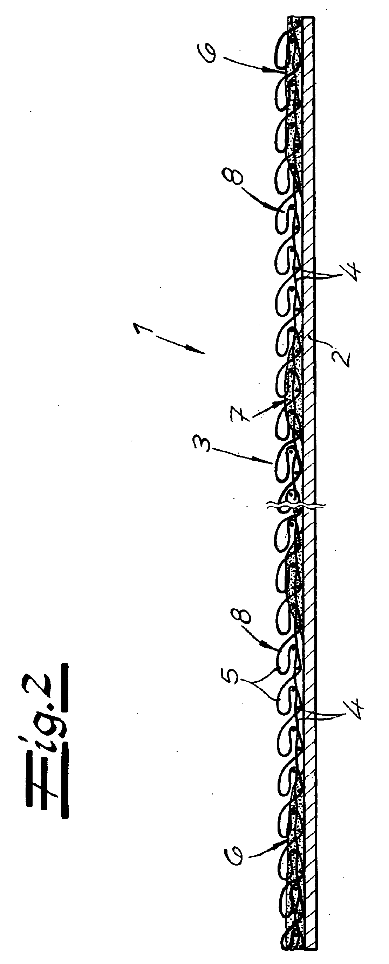

Das in den Fig. 1 und 2 schematisch dargestellte Verbundstoffelement 1 ist als weiblicher Teil eines Klettverschlusses einsetzbar. Der Verbundstoff ist insbesondere für Klettverschlüsse an Wegwerfprodukten, z. B. Babywindeln, bestimmt. Er besteht aus einer Trägerfolie 2 und einem auf die Trägerfolie aufkaschierten textilen Material 3, welches eine zur Verbindung mit Kletthaken geeignete Oberflächenstruktur aufweist. Bei dem in Fig. 1 und 2 dargestellten Material handelt es sich um eine Kettwirkware mit einem Grundgelege aus Filamentgarnen 4 sowie mit dem Grundgelege wirktechnisch verbundene Schlaufen 5. Die Schlaufen 5 sind oberflächlich angeordnet und zur Verbindung mit nicht dargestellten Kletthaken eines Verschlussbandes geeignet.

Die Trägerfolie 2 und das textile Material 3 sind nicht vollflächig verbunden. Eine flächige Verbindung entlang des Randes des Verbundstoffelementes 1 mit einer Breite zwischen 5 mm und 30 mm bildet einen Klebstoffrahmen 6 und weist ein innenliegendes Klebstoffmuster 7 mit klebstofffreien Bereichen 8 auf.

Der Anteil der Klebstofffläche innerhalb des von dem Klebstoffrahmen 6 umschlossenen Bereiches beträgt 20 % bis 80 % bezogen auf die von dem Klebstoffrahmen 6 umschlossene Fläche. An den Klebeflächen ist das textile Material durch Klebstoffauftrag fest an der Trägerfolie 2 verankert. Der Fig. 2 entnimmt man, dass die Filamentgarne 4 des textilen Materiales 3 im Klebstoff, beispielsweise einem Schmelzklebstoff, eingesunken sind und von diesem umschlossen werden. Dabei kann in Kauf genommen werden, dass die Schlaufen 5 im Bereich der Klebeflächen teilweise mitverklebt werden und im Bereich der Klebeflächen nur wenig wirksam sind. In den klebstofffreien Bereichen 8 liegen die Trägerfolie 2 und das textile Material 3 lose aufeinander. Kletthaken lassen sich in den klebstofffreien Bereichen 8 des Verbundstoffes tief in das textile Material 3 einschieben, wobei nicht nur eine Verhakung an den oberflächlichen freien Schlaufen 5 erfolgt, sondern zusätzlich auch die Filamentgarne 4 des Grundgeleges für die Verankerung der Kletthaken zur Verfügung stehen. So können die Kletthaken zum Teil hinter die Filamentgarne 4 des textilen Materials 3 greifen. Die klebstofffreien Bereiche 8 des Verbundstoffes zeichnen sich durch eine große Klettwirkung aus und verleihen dem Verbundstoff gute Gebrauchseigenschaften. Durch den Klebstoffrahmen 6 am Rand des Verbundstoffelements 1 kann auch bei einer mehrfachen Betätigung des Klettverschlusses ein Zerfransen des Randes der textilen Schicht oder gar ein Abreißen der textilen Schicht von der Trägerfolie 2 verhindert werden.

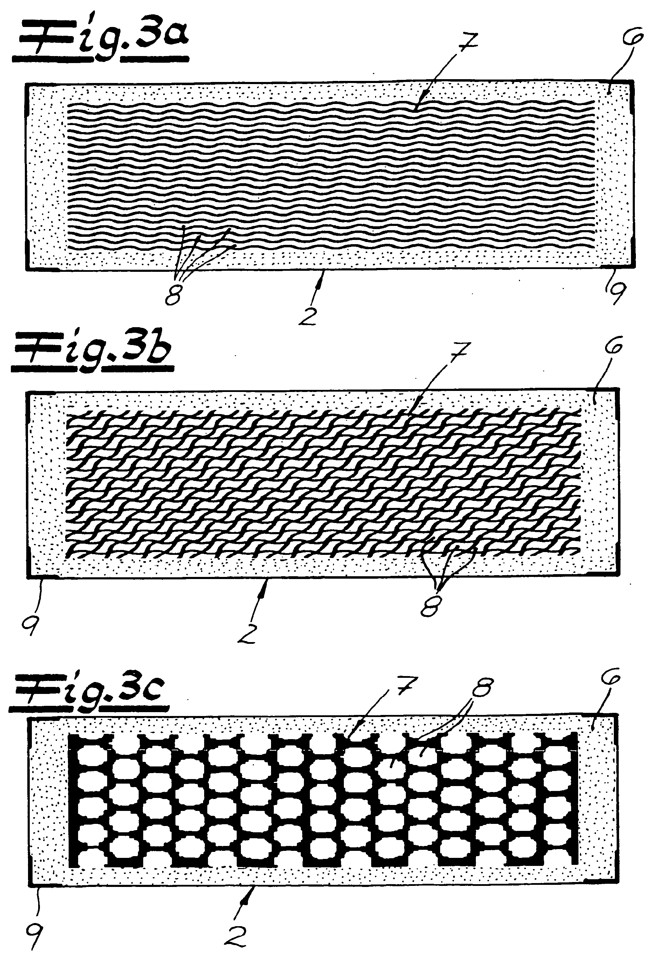

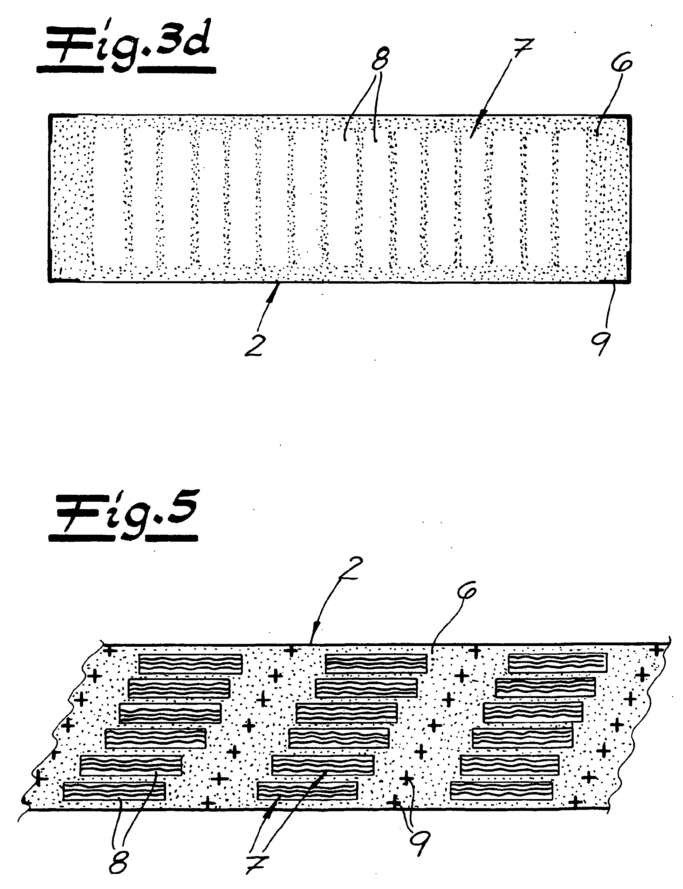

Die Fig. 3a bis 3d zeigen verschiedene Klebstoffmuster 7 innerhalb des Klebstoffrahmens 6. In dem Ausführungsbeispiel 3a bildet der Klebstoff innerhalb des Klebstoffrahmens 6 ein Muster aus parallelen, wellenförmig verlaufenden Streifen. Im Ausführungsbeispiel der Fig. 3b bildet der Klebstoff innerhalb des Klebstoffrahmens 6 ein Muster aus sich kreuzenden Streifen. Die Fig. 3c zeigt innerhalb des Klebstoffrahmens 6 ein Muster mit einer zellenförmigen Struktur, wobei die zellenförmige Struktur geschlossene Zellen mit klebstofffreien Bereichen 8 aufweist. Fig. 3d zeigt innerhalb des Klebstoffrahmens 6 eine Struktur, die aus gradlinigen Streifen besteht, die parallel zu den kurzen Seiten des rechteckigen Verbundstoffelements ausgerichtet sind. In allen Ausführungsbeispielen sollten die Klebeflächen innerhalb der Klebstoffrahmen 6 nicht weniger als 20 % und maximal 80 % der Fläche betragen.

Das textile Material 3 weist vorzugsweise ein Flächengewicht zwischen 5 g/m² und 50 g/m² auf. Es ist luftdurchlässig und durchscheinend. Deshalb kann die Trägerfolie 2 mit einem Dekormuster und/oder Rapportmarken 9 zur Markierung der Klebstoffrahmen 6 bedruckt werden. Als Trägerfolien 2 sind Folien z. B. aus Polyolefinen, Polyester, Polyamid, Mischungen oder Copolymerisaten dieser Polymere einsetzbar. Sie weisen vorzugsweise ebenfalls ein Flächengewicht zwischen 5 g/m² und 50 g/m² auf.

Zur Verklebung der Trägerfolie 2 mit dem textilen Material 3 können beispielsweise Schmelzklebstoffe auf der Basis PAO, EVA, SBS, SIS, reaktive Polyurethanklebstoffe, Acrylatklebstoffe sowie auch strahlenhärtende Klebstoffe eingesetzt werden. Die Klebstoffmengen werden auf die Klebefläche so abgestimmt, dass ein fester Verbund zwischen Trägerfolie 2 und textilem Material 3 gewährleistet ist. Daher sind die Klebstoffauftragsmengen anwendungsabhängig variabel festlegbar.

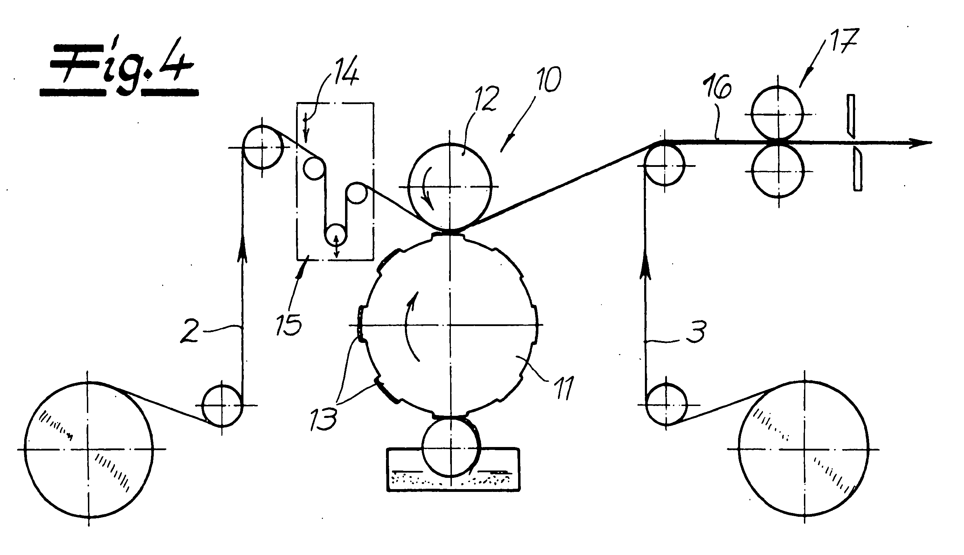

Ein Verfahren zur Herstellung der Verbundstoffelelemente 1 ist in Fig. 4 schematisch dargestellt. Auf eine Trägerfolie 2 wird Klebstoff in einem Muster appliziert, welches sich aus Klebeflächen und klebstofffreien Bereichen 8 zusammensetzt. Der Klebstoff kann durch Düsenauftragsverfahren, durch eine Sprühtechnik und ähnliche Verfahren auf die Trägerfolie 2 aufgebracht werden. Bei der in den Ausführungsbeispielen dargestellten bevorzugten Ausführung des Verfahrens wird der Klebstoff nach einem Rotationsdruckverfahren auf die Trägerfolie 2 aufgebracht. Hierbei durchläuft die Trägerfolie eine Druckwalzenanordnung 10 bestehend aus einem Gravurzylinder 11 und einem die Trägerfolie 2 an den Gravurzylinder 11 andrückenden Gegenzylinder 12, wobei die Oberfläche des Gravurzylinders 11 mit einer dem Klebstoffmuster entsprechenden Gravur 13 versehen ist. Bei der dargestellten Ausgestaltung wird auf die Oberfläche des Gravurzylinders 11 ein Klebstoff aufgebracht, der an den erhabenen Flächen der Gravur 13 auf die Trägerfolie übertragen wird. Um die Klebstoffrahmen 6 bezüglich Rapportmarken 9, die in dem gewählten Ausführungsbeispiel bereits auf die Folienbahn gedruckt sind, zu positionieren, wird mit einer Leseeinheit 14 die Position der Rapportmarken 9 bezüglich des Drehwinkels des Gravurzylinders 11 bestimmt. Mit einem verstellbaren System von Umlenkrollen 15 wird dann der Vorlauf der Folienbahn vor dem Gravurzylinder 11 so eingestellt, dass die Klebstoffrahmen 6 an einer bezüglich der Rapportmarken 9 vorgegebenen Position auf die Trägerfolie 2 aufgebracht werden. Anschließend wird eine Bahn aus textilem Material 3, welches eine zur Verbindung mit Kletthaken geeignete Oberflächenstruktur aufweist, auf die mit Klebstoff versehene Seite der Trägerfolie 2 aufgebracht. Die dabei gebildete zweilagige Bahn 16 durchläuft den Walzenspalt eines Walzenpaares 17, in dem die Trägerfolie mit dem textilen Material verpresst wird. Schließlich werden aus dem Verbundstoff Verbundstoffelemente abgetrennt. Für ein rapportgenaues Abtrennen können die auf der Trägerfolie aufgedruckten Rapportmarken 9 genutzt werden. Das Abtrennen muss nicht direkt nach dem Kaschierprozess erfolgen, sondern kann auch erst unmittelbar vor der weiteren Verarbeitung, z. B. im Zuge der Windelherstellung durchgeführt werden. Der Verbundstoff wird in diesem Fall zweckmäßig als Rollenware verarbeitet. Die als Klebstoffrahmen 6 bezeichneten Klebeflächen führen zu einer geringeren Staubbildung beim Schneiden des Verbundstoffes. Da der Schnitt jeweils in den flächig verklebten Zonen erfolgt, entstehen keine losen Faserreste und Filamentreste, die als Staub die Anlage verschmutzen könnten.

## Patentansprüche

1. Verbundstoffelement (1) für einen Klettverschluss, mit einer Trägerfolie (2) und einem auf die Trägerfolie (2) aufkaschierten textilen Material (3), welches eine zur Verbindung mit Kletthaken geeignete Oberflächenstruktur aufweist, wobei die Trägerfolie (2) und das textile Material (3) in einem Randbereich, der sich entlang der Ränder des Verbundstoffelementes erstreckt und die Form eines Rahmens (6) aufweist, verbunden sind und wobei im Bereich innerhalb dieses Rahmens (6) eine nicht vollflächige Verbindung zwischen der Trägerfolie (2) und dem textilen Material (3) vorgesehen ist, **dadurch gekennzeichnet, dass** die Trägerfolie (2) und das textile Material (3) miteinander verklebt sind, wobei der Klebstoff zwischen der Trägerfolie (2) und dem textilen Material (3) in dem rahmenförmigen Randbereich flächig aufgetragen ist und einen Klebstoffrahmen (6) bildet und wobei der Klebstoff in dem von dem Klebstoffrahmen (6) umschlossenen Bereich in einem Klebstoffmuster (7) angeordnet ist, welches sich aus Klebeflächen und klebstofffreien Bereichen (8) zusammensetzt.

2. Verbundstoffelement nach Anspruch 1, **dadurch gekennzeichnet, dass** der Anteil der Klebstofffläche innerhalb des von dem Klebstoffrahmen (6) umschlossenen Bereiches 20 % bis 80 % bezogen auf die vom Klebstoffrahmen (6) umschlossene Fläche beträgt.

3. Verbundstoffelement nach Anspruch 1 oder 2, **dadurch gekennzeichnet, dass** die Trägerfolie (2) und das textile Material (3) in dem von den Klebstoffrahmen umschlossenen Bereich punktförmig verklebt sind.

4. Verbundstoffelement nach Anspruch 1 oder 2, **dadurch gekennzeichnet, dass** der Klebstoff innerhalb des Klebstoffrahmens (6) ein Muster aus parallelen oder sich kreuzenden Streifen bildet.

5. Verbundstoffelement nach Anspruch 1 oder 2, **dadurch gekennzeichnet, dass** der Klebstoff innerhalb des Klebstoffrahmens (6) ein Muster mit einer zellenförmigen Struktur bildet.

6. Verbundstoffelement nach einem der Ansprüche 1 bis 5, **dadurch gekennzeichnet, dass** der flächig verbundene Randbereich eine Breite von 5 mm bis 30 mm aufweist.

7. Verbundstoffelement nach einem der Ansprüche 1 bis 6, **dadurch gekennzeichnet, dass** der Bereich innerhalb des rahmenförmigen Randbereiches eine Länge zwischen 20 und 100 mm und eine Breite zwischen 50 und 500 mm aufweist.

8. Verbundstoffelement nach einem der Ansprüche 1 bis 7, **dadurch gekennzeichnet, dass** das textile Material (3) aus einer Kettwirkware besteht, welches ein Grundgelege aus Filamentgarnen (4) und mit dem Grundgelege wirktechnisch verbundene Schlaufen (5) aufweist.

9. Verbundstoffelement nach einem der Ansprüche 1 bis 8, **dadurch gekennzeichnet, dass** das textile Material (3) ein Flächengewicht von 5 g/m² bis 50 g/m² aufweist.

10. Verbundstoffelement nach einem der Ansprüche 1 bis 9, **dadurch gekennzeichnet, dass** die Trägerfolie (2) bedruckt und/oder geprägt ist.

11. Verfahren zur Herstellung eines Verbundstoffelementes (1) nach einem der Ansprüche 1 bis 10 mit folgenden Verfahrensschritten:
- auf eine Folienbahn (2) wird eine Klebstofffläche im Rapport appliziert, wobei des Klebstoff in einem von einem Klebstoffrahmen (6) umschlossenen Bereich in einem Klebstoffmuster (7) angeordnet ist, welches sich aus Klebeflächen und Klebstofffreien Bereichen (8) zusammensetzt;
- eine Materialbahn aus einem textilen Material (3), welches eine zur Verbindung mit Kletthaken geeignete Oberflächenstruktur aufweist, wird auf die mit Klebstoff versehene Seite der Folienbahn (2) aufgebracht und mit dieser zu einem Verbundstoff verpresst;
- aus dem Verbundstoff werden Verbundstoffelemente (1) abgetrennt, die entlang der Ränder flächig durch die als Klebstoffrahmen (6) applizierten Klebeflächen verbunden sind.

12. Verfahren nach Anspruch 11, **dadurch gekennzeichnet, dass** der Klebstoff im Rotationsdruckverfahren auf die Folienbahn aufgebracht wird.

13. Verfahren nach Anspruch 11 oder 12, **dadurch gekennzeichnet, dass** auf die Folienbahn sichtbare oder nur unter UV-Licht sichtbare Rapportmarken (9) zur Markierung der Klebstoffrahmen (6) gedruckt und durch Ansteuerung dieser Rapportmarken (9) die Klebstoffelemente rapportgenau von der Materialbahn abgetrennt werden.

## Claims

1. A composite material element (1) for a hook-and-loop fastener comprising a carrier film (2) and a textile material (3), which has a surface structure suitable for a connection with fastener hooks, laminated onto the carrier film (2), wherein the carrier film (2) and the textile material (3) are connected in a periphery extending along the edges of the composite material element and having the form of a frame (6), and wherein in the region within this frame (6), a non-full surface connection between the carrier film (2) and the textile material (3) is provided, **characterized in that** the carrier film (2) and the textile material (3) are glued together, wherein the adhesive between the carrier film (2) and the textile material (3) is applied over the surface within the frame-shaped periphery and forms an adhesive frame (6), and wherein the adhesive is arranged in the region enclosed by the adhesive frame (6) in an adhesive pattern (7) consisting of adhesive areas and adhesive-free areas (8).

2. The composite material element according to claim 1, **characterized in that** the portion of the adhesive area within the region enclosed by the adhesive frame (6) is 20 % to 80 % with respect to the area enclosed by the adhesive frame (6).

3. The composite material element according to claim 1 or claim 2, **characterized in that** the carrier film (2) and the textile material (3) are glued at points within the region enclosed by the adhesive frame.

4. The composite material element according to claim 1 or claim 2, **characterized in that** the adhesive within the adhesive frame (6) forms a pattern of parallel or intersecting strips.

5. The composite material element according to claim 1 or claim 2, **characterized in that** the adhesive within the adhesive frame (6) forms a pattern having a cellular structure.

6. The composite material element according to any one of the claims 1 to 5, **characterized in that** the periphery connected over the surface has a width of 5 mm to 30 mm.

7. The composite material element according to any one of the claims 1 to 6, **characterized in that** the region within the frame-shaped periphery has a length between 20 and 100 mm and a width between 50 and 500 mm.

8. The composite material element according to any one of the claims 1 to 7, **characterized in that** the textile material (3) consists of a warp-knitted fabric comprising a basic interlaid scrim of filament yarns (4) and loops (5) connected to the basic interlaid scrim by means of knitting technology.

9. The composite material element according to any one of the claims 1 to 8, **characterized in that** the textile material (3) has a basic weight of 5 g/m² to 50 g/m².

10. The composite material element according to any one of the claims 1 to 9, **characterized in that** the carrier film (2) is printed and/or embossed.

11. A method for manufacturing a composite material element (1) according to any one of the claims 1 to 10, comprising the following process steps:
- onto a film web (2) an adhesive area is applied in a repeating pattern, wherein the adhesive is arranged in a region enclosed by an adhesive frame (6) in an adhesive pattern (7), consisting of adhesive areas and adhesive-free areas (8);
- a material web of a textile material (3) comprising a surface structure suitable for a connection with fastener hooks is applied onto the side of the film web (2) provided with adhesive, and is pressed together with the same to form a composite material;
- from the composite material, composite material elements (1) are cut off, which are connected over the surface along the edges by means of the adhesive areas applied as adhesive frames (6).

12. The method according to claim 11, **characterized in that** the adhesive is applied onto the film web by means of the rotary printing method.

13. The method according to claim 11 or claim 12, **characterized in that** onto the film web, repeating pattern marks (9), which are visible or visible only under UV light, are printed for marking the adhesive frames (6), and by controlling these repeating pattern marks (9), the adhesive elements are cut off from the material web with repeating position accuracy.

## Revendications

1. Élément de tissu composite (1) pour une fermeture velcro, comprenant un film support (2) et un matériau (3) textile contrecollé sur le film support (2), qui présente une structure de surface appropriée pour la liaison avec des crochets velcro, le film support (2) et le matériau (3) textile étant reliés dans une zone périphérique, qui s'étend le long des bords du élément de tissu composite et présente la forme d'un cadre (6) et une liaison, qui n'est pas sur toute la surface, entre le film support (2) et le matériau (3) textile étant prévue dans la zone à l'intérieur de ce cadre (6), **caractérisé en ce que** le film support (2) et le matériau (3) textile sont collés l'un à l'autre, la colle étant appliquée en surface entre le film support (2) et le matériau (3) textile dans la zone périphérique en forme de cadre et formant un cadre de colle (6) et la colle dans la zone entourée par le cadre de colle (6) étant disposée dans un modèle de colle (7), lequel se compose de surfaces adhésives et de zones (8) sans colle.

2. Élément de tissu composite selon la revendication 1, **caractérisé en ce que** la fraction de la surface de colle à l'intérieur de la zone entourée par le cadre de colle (6) représente 20 % à 80 % par rapport à la surface entourée par le cadre de colle (6).

3. Élément de tissu composite selon la revendication 1 ou 2, **caractérisé en ce que** le film de support (2) et le matériau (3) textile sont collés de façon ponctuelle dans la zone entourée par le cadre de colle.

4. Élément de tissu composite selon la revendication 1 ou 2, **caractérisé en ce que** la colle forme à l'intérieur du cadre de colle (6) un modèle constitué de bandes parallèles ou de bandes qui se croisent.

5. Élément de tissu composite selon la revendication 1 ou 2, **caractérisé en ce que** la colle forme à l'intérieur du cadre de colle (6) un modèle avec une structure en forme de cellule.

6. Élément de tissu composite selon l'une quelconque des revendications 1 à 5, **caractérisé en ce que** la zone périphérique reliée en surface présente une largeur de 5 mm à 30 mm.

7. Élément de tissu composite selon l'une quelconque des revendications 1 à 6, **caractérisé en ce que** la zone à l'intérieur de la zone périphérique en forme de cadre a une longueur comprise entre 20 et 100 mm et une largeur comprise entre 50 et 500 mm.

8. Élément de tissu composite selon l'une quelconque des revendications 1 à 7, **caractérisé en ce que** le matériau (3) textile est à base d'un article tricoté en chaîne, qui présente une structure de base composé de fils de filament (4) et des boucles (5) reliés par tricotage à la structure de base.

9. Élément de tissu composite selon l'une quelconque des revendications 1 à 8, **caractérisé en ce que** le matériau (3) textile présente un poids surfacique de 5 g/m² à 50 g/m².

10. Elément de tissu composite selon l'une quelconque des revendications 1 à 9, **caractérisé en ce que** le film support (2) est imprimé et/ou gaufré.

11. Procédé pour la fabrication d'un élément de tissu composite (1) selon l'une quelconque des revendications 1 à 10 comprenant les étapes de procédé suivantes :
- sur une bande de film (2) est appliquée une surface de colle dans le rapport, la colle étant disposée dans une zone entourée par un cadre de colle (6) dans un modèle de colle (7) qui se compose de surfaces adhésives et de zones (8) sans colle ;
- une bande de matériau à base d'un matériau (3) textile, qui présente une structure de surface appropriée pour la liaison avec des crochets velcro, est appliquée sur le côté, doté de colle, de la bande de film (2) et est comprimée avec celle-ci pour former un tissu composite ;
- des éléments de tissu composites (1) sont séparés du tissu composite (1), qui sont reliés en surface le long des bords par les surfaces adhésives appliquées comme cadre de colle (6).

12. Procédé selon la revendication 11, **caractérisé en ce que** la colle est appliquée dans le procédé d'impression par rotative sur la bande de film.

13. Procédé selon la revendication 11 ou 12, **caractérisé en ce que** des repères de rapport (9) visibles ou visibles seulement à la lumière UV sont imprimés sur la bande de film pour le marquage des cadres de colle (6) et les éléments de colle sont séparés de façon précise au niveau du rapport de la bande de matériau par direction sur ces repères de rapport (9).
